# EUROPÄISCHE PATENTSCHRIFT

(11) **EP 0 285 962 B1**
(45) Veröffentlichungstag und Bekanntmachung des Hinweises auf die Patenterteilung: **17.02.1993**
(21) Anmeldenummer: 88105013.2
(22) Anmeldetag: 28.03.1988
(51) Int. Cl.: A61B 17/39

(54) **Überwachungsschaltung für ein HF-Chirurgiegerät**
Control circuit for a HF chirurgical apparatus
Circuit de contrôle pour un appareil chirurgical à haute fréquence

(30) Priorität: 10.04.1987 DE 3711702
(43) Veröffentlichungstag der Anmeldung: 12.10.1988
(73) Patentinhaber: SIEMENS AKTIENGESELLSCHAFT, 80333 München (DE)
(72) Erfinder: Feucht, Peter, Dipl.-Ing., D-1000 Berlin 62 (DE)

(56) Entgegenhaltungen:
- DE-A- 3 502 193
- DE-A- 3 623 688

## Beschreibung

Die Erfindung bezieht sich auf eine Überwachungsschaltung nach dem Oberbegriff des Patentanspruches 1.

Auf dem Gebiet der HF-Chirurgie sind Überwachungsschaltungen verschiedener Art bekannt (Siemens-Prospekt "Radiotom 804"). Das Ziel der meisten dieser Überwachungsschaltungen ist es, den Patienten beim Schneiden und/oder Koagulieren vor unerwünschten Verbrennungen zu schützen.

Die "Medizingeräteverordnung" (Deutsches Bundesgesetzblatt, Jahrgang 1985, Teil I, Seiten 93 bis 98) schreibt in § 3 vor, daß bei einem medizinisch-technischen Gerät sichergestellt sein muß, daß Patienten, Beschäftigte oder Dritte bei der bestimmungsgemäßen Verwendung des Geräts gegen Gefahren für Leben und Gesundheit soweit geschützt sind, wie es die Art der bestimmungsgemäßen Verwendung gestattet. Weiterhin ist vorgeschrieben, daß HF-Chirurgiegeräte mit einer Warneinrichtung für den Fall einer gerätebedingten Fehldosierung ausgerüstet sein müssen. Entsprechende Bestimmungen finden sich in der IEC 601, Teil I, § 51. Ziel der vorliegenden Erfindung ist es, eine Überwachungsschaltung anzugeben, die diesen Anforderungen genügt.

Von besonderem Interesse ist bei einem HF-Chirurgiegerät der sog. "erste Fehlerfall". Hiermit ist das Auftreten eines ersten gerätebedingten Fehlers gemeint; in einem solchen Fall sollte die jeweils eingestellte Leistung (z.B. 100 Watt), die geringer sein kann als die maximal abgebbare Leistung (z.B. 400 Watt), gar nicht oder nur um einen bestimmten Toleranzwert (z.B. 30%) überschritten werden. Im angegebenen Beispiel dürfte also beim Auftreten eines Fehlers, z. B. in der Leistungserzeugungseinrichtung oder in der Steuerungseinrichtung infolge Ausfalls eines Transistors, die abgegebene Leistung 130 Watt nicht überschreiten.

Aus der DE-A-35 02 193 ist eine Überwachungsschaltung der eingangs genannten Art bekannt. Mit dieser Überwachungsschaltung werden periodisch die Spannung und der Strom an zum Patienten führenden Plattenelektroden nur überwacht. Daraus wird die an den Patienten abgegebene Leistung mit einem Mikrocomputer errechnet. Dazu sind A/D-Wandler nötig. Diese Überwachungsschaltung erzeugt nur aus den während der Überwachungsperioden (Abtastperioden) vorliegenden Strom- und Spannungswerten einen rechnerischen Leistungswert. Eine kontinuierliche Überwachung innerhalb der Behandlungsdauer des Patienten ist sonach nicht möglich. Die bekannte Überwachungsschaltung weist daher Überwachungslücken auf. Die Sicherheit (Genauigkeit) in einem insbesondere kurzzeitigen Fehlerfall ist sonach eingeschränkt.

Aus der DE-A-36 23 688 ist ein HF-Chirurgiegerät mit einer Überwachungs- und Steuerschaltung bekannt, bei der an einem ersten Ausgang einer HF-Leistungsstufe ein von anschließbaren Patientenelektroden gesonderter Strompfad angeschlossen ist, in welchem ein Lastwiderstand über einen An-/Ausschalter mit einem gegenphasigen Ausgang der HF-Leistungsstufe verbindbar ist. Im Falle einer solchen Verbindung (Prüfbetrieb) wird über einen Stromdetektor der Stromfluß in dem Lastwiderstand erfaßt. Folglich kann während des Prüfbetriebes nur der Stromfluß gemessen werden. Eine Messung der Leistung oder des an einem Patienten während der Behandlung abgegebenen Stromes ist mit dieser Überwachungsschaltung nicht möglich.

Der Erfindung liegt die Aufgabe zugrunde, eine Überwachungsschaltung für ein HF-Chirurgiegerät anzugeben, mit der der sogenannte erste Fehlerfall mit verbesserter Sicherheit (Genauigkeit) erkannt werden kann.

Diese Aufgabe wird erfindungsgemäß durch die im Patentanspruch 1 genannten Merkmale gelöst. Mit der erfindungsgemäßen Überwachungsschaltung kann während des Arbeitens mit dem HF-Chirurgiegerät an einem Patienten die tatsächlich an diesen Patienten abgegebene Leistung mittels eines Leistungsmessers gemessen werden. Des weiteren kann vor dem Arbeiten an dem Patienten mittels eines Lastwiderstandes die abgegebene Leistung gemessen werden. In jedem Fall wird erfindungsgemäß die tatsächlich abgegebene Leistung mit nur einer Meßschaltung gemessen. Das erhöht die Meßsicherheit (Genauigkeit). Jeder gemessene Leistungs-Istwert wird mit einem Leistungs-Sollwert verglichen. Liegt eine Abweichung zwischen dem vorgegebenen Leistungs-Sollwert und dem gemessenen Leistungs-Istwert vor, so kann ein Ausgangssignal zur Freigabe für einen Alarm oder für die Abschaltung des HF-Chirurgiegeräts erzeugt werden.

Prinzipiell könnte man auch so vorgehen: Man verwendet ein HF-Chirurgiegerät mit einer HF-Leistungsstufe, die über ein Einstellorgan in der Leistung eingestellt wird. Man verwendet weiter eine Anzeigeeinrichtung, die die wirklich abgegebene Leistung während des Arbeitens mit dem HF-Chirurgiegerät mißt. Man verwendet weiterhin einen Vergleicher, der ständig die abgegebene mit der eingestellten HF-Leistung vergleicht und beim Überschreiten der eingestellten Leistung einen Alarm auslöst und/oder das HF-Chirurgiegerät automatisch abschaltet. Eine solche Leistungs-Überwachungsschaltung ist sicher zweckmäßig. Beim Einschalten hat sie jedoch den Nachteil, daß der Patient über die neutrale und aktive Elektrode in den Überwachungskreis eingeschlossen ist und daher bei Vorliegen eines Defekts gefährdet sein könnte. Die erwähnte Anwendung des Lastwiderstands im Ausgangskreis der HF-Leistungsstufe vermeidet diesen Nachteil.

Vorteilhafte Ausführungsbeispiele der Erfindung sind in den Unteransprüchen näher charakterisiert und werden im folgenden anhand von zwei Figuren näher erläutert. Es zeigen:
- Fig. 1: ein HF-Chirurgiegerät mit einer Überwachungsschaltung für die abgegebene Leistung und
- Fig. 2: ein Widerstands-Leistungs-Diagramm.

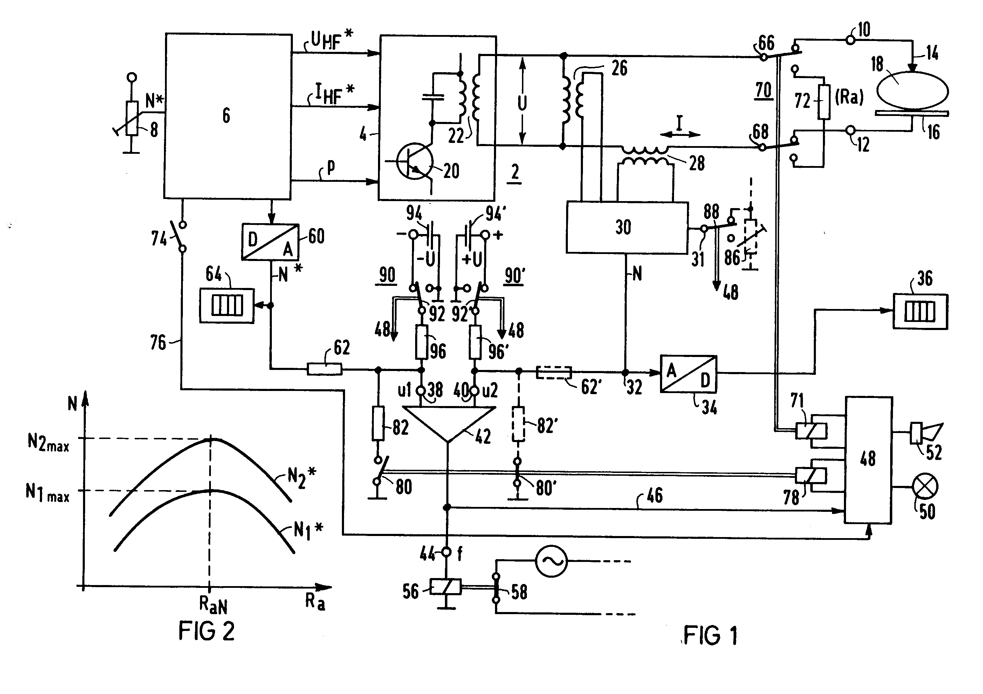

Nach Fig. 1 enthält ein HF-Chirurgiegerät 2 im wesentlichen eine HF-Leistungsstufe 4 und eine Steuerschaltung 6 für deren Ansteuerung. Die Steuerschaltung 6 ist mit einem Einstellorgan 8 zum Einstellen des Soll-Werts N* der abgegebenen HF-Leistung ausgerüstet. Weiterhin enthält das HF-Chirurgiegerät 2 zwei Ausgangsklemmen 10, 12, an die eine aktive bzw. neutrale Elektrode 14 bzw. 16 zum Schneiden und/oder Koagulieren anschließbar sind. Der zu behandelnde Patient ist mit 18 bezeichnet.

Die maximal aus der HF-Leistungsstufe 4 entnehmbare Leistung Nₘₐₓ ist durch gewisse Parameter bestimmt. Zu diesen zählen z.B. die Betriebsspannung, der Strom, die Ansteuerung, der Wirkungsgrad und die Impulsform der Ansteuerung der HF-Leistungsstufe 4. Es wird vorliegend generell angenommen, daß sich einige dieser Parameter mit Hilfe des Einstellorgans 8 beeinflussen lassen und daß somit eine vorgegebene HF-Leistung N* einstellbar ist. Beispielsweise kann es sich bei Steuerschaltung 6 um eine digitale Steuerschaltung handeln, die Digitalwerte für den Soll-Wert der HF-Spannung U_{HF}*, für den HF-Strom I_{HF}* und eine Impulsfolge p zur Ansteuerung von Schaltelementen 20 innerhalb der HF-Leistunsstufe 4 vorgibt. Gemäß den so vorgegebenen digitalen Werten U_{HF}*, I_{HF}* und p wird vom Leistungs- oder Ausgangstransformator 22 an die Ausgangsklemmen 10, 12 eine Leistung N geliefert, die dem Soll-Wert N* entspricht, falls kein Fehler vorliegt.

Im vorliegenden Ausführungsbeispiel ist vorgesehen, daß die HF-Leistungsstufe 4 über eine Anzahl von Transistoren, von denen nur der Transistor 20 gezeigt ist, und einen Leistungs-Transformator 22 ein HF-Chirurgie-Signal zum Schneiden und/oder Koagulieren erzeugt, das erdfrei über die Ausgangsklemmen 10, 12 entnommen wird.

Mit Hilfe eines ersten Transformators 26 wird potentialfrei ein Bruchteil der HF-Spannung U zwischen den Ausgangsleitungen der HF-Leistungsstufe 4 erfaßt. Mit einem zweiten Transformator 28 wird analog ebenfalls potentialfrei ein Äquivalent des HF-Stroms I in der einen Ausgangsleitung gemessen. Die Ergebnisse der Spannungsmessung aus dem Transformator 26 und der Strommessung aus dem Transformator 28 werden in eine Watt-Meter-Schaltung oder in einen Leistungsmesser 30 gegeben, und zwar zur Ermittlung der (hier über die Ausgangsklemmen 10, 12) tatsächlich abgegebenen Wirkleistung. Der Leistungsmesser 30 ist vorzugsweise ein Multiplizierer und arbeitet auf der Basis phasengetreuer Multiplikation. Solche Multiplizierglieder sind als integrierte Schaltkreise heutzutage relativ preiswert erhältlich, und die Ergebnisse sind mit Fehlern unter 10 % behaftet.

Das Ergebnis der Leistungsmessung ist der Ist-Wert N der abgegebenen HF-Leistung oder diesem proportional. Dieser Ist-Wert N wird über einen Verzweigungspunkt 32 einem Analog-Digital-Wandler 34 und von dort einem Gerät zur Ziffernanzeige oder einer Anzeigeeinrichtung 36 zugeführt. Hier kann der jeweils aktuelle Ist-Wert N der über die Ausgangsklemmen 10, 12 abgegebenen Wirkleistung vom Operateur abgelesen werden.

Vom Verzweigungspunkt 32 aus wird das Ergebnis N der Leistungsmessung auch dem zweiten Eingang 40 eines Komparators 42 zugeführt. Das dem Ist-Wert N entsprechende Eingangssignal ist dabei mit u2 bezeichnet. Dieses Eingangssignal u2 wird im Komparator 42 mit einem ersten Eingangssignal u1, das dem ersten Eingang 38 zugeführt ist, verglichen. Das erste Eingangssignal u1 ist der Soll-Wert N* oder diesem proportional. Für den Fall, daß das zweite Eingangssignal u2 das erste Eingangssignal u1 übersteigt, d.h. für den beschriebenen Fall, daß die abgegebene Leistung N den Soll-Wert N* übersteigt, ggf. mit einer gewissen Toleranz, wird am Ausgang 44 des Komparators 42 ein Freigabesignal f entnommen. Mit diesem Freigabesignal f kann der Arzt (Operateur) durch einen Alarm gewarnt werden. Dies ist durch eine Verbindungsleitung 46 zu einem Testgerät 48 und zwei Alarmeinheiten in Form einer optischen Signaleinrichtung 50 und einer akustischen Signaleinheit 52 symbolisiert. Das Freigabesignal f kann andererseits aber auch zum Abschalten des HF-Chirurgiegeräts 2 führen. Dazu ist der Ausgang 44 mit einem Relais 56 verbunden, das auf einen Schalter 58 einwirkt, welcher die Spannungsversorgung des HF-Chirurgiegeräts 2 ein- und ausschaltet.

Der am Einstellorgan 8 eingestellte Soll-Wert N* wird der Steuerschaltung 6 als digitaler Wert entnommen und in einem Digital-Analog-Wandler 60 zum analogen Soll-Wert N* gewandelt. Dieser gelangt über einen Widerstand 62 und den ersten Eingang 38 in den Komparator 42. Der analoge Soll-Wert N* wird außerdem in einem Anzeigebaustein oder einer Anzeigeeinrichtung 64 ständig zur Anzeige gebracht.

Im Normalbetrieb (im Gegensatz zu dem im folgenden geschilderten Testbetrieb) kann der Operateur sich durch einen Blick auf die Anzeigeeinheiten 36, 64 überzeugen, ob Ist- und Soll-Wert N, N* der HF-Leistung übereinstimmen.

Die gezeigte Überwachungsschaltung hat noch eine weitere Besonderheit, durch die ein Testbetrieb ermöglicht wird. Nachdem der Ist-Wert N der HF-Leistung mit Hilfe der Transformatoren 26, 28 erfaßt ist, wird das HF-Chirurgie-Signal den Kontakten 66, 68 eines generell mit 70 bezeichneten Umschalters zugeführt. Dieser Umschalter 70 umfaßt vorzugsweise die Kontakte eines Relais 71, das von der Testschaltung 48 angesteuert wird. Die Ansteuerung ist durch zweifache Verbindungslinien verdeutlicht.

Normalerweise wird das HF-Chirurgie-Signal vom Ausgang der HF-Leistungsstufe 4 zu den Ausgangsklemmen 10, 12 noch über weitere Schaltungen, die dem Stand der Technik entsprechen und hier nicht wiedergegeben sind, zu den Elektroden 14, 16 der HF-Chirurgie geführt.

Mit Hilfe des Umschalters 70 kann an den Ausgang der HF-Leistungsstufe 4 ein Lastwiderstand 72 mit vorgegebenem Widerstandswert Rₐ angeschaltet werden, und zwar anstelle der Elektroden 14, 16. Nach dem Umschalten kann die bereits geschilderte Einrichtung 26, 28, 30, 42, 60 verwendet werden, um zu überprüfen, ob der nun vorhandene Ist-Wert N nennenswert vom Sollwert N* abweicht. Mit anderen Worten: Mit Hilfe des Relais 71 kann von der Testschaltung 48 zu bestimmten Zeitpunkten ein HF-Leistungstest ("Selbsttest") durchgeführt werden. Ein solcher Selbsttest wird durch Betätigung eines Testschalters 74 eingeleitet, der in einer Verbindungsleitung 76 zwischen der Steuerschaltung 6 und der Testschaltung 48 liegt. Mit Hilfe dieses Testschalters 74 wird also die Testschaltung 48 zum Ansprechen des Relais 71 gebracht. Nach dem Ansprechen ist die Klemme 66 mit dem einen Ende und die Klemme 68 mit dem anderen Ende des Lastwiderstands 72 verbunden. Damit ist also der Lastwiderstand 72 anstelle der HF-Chirurgie-Elektroden 14, 16 angeschlossen.

Mit Hilfe dieses Lastwiderstands 72 wird beim Selbsttest die abgegebene HF-Leistung, das ist vorzugsweise die Maximalleistung Nₘₐₓ (vgl. Fig. 2), erfaßt.

Nach Fig. 2 ist der Widerstandswert Rₐ des Lastwiderstandes 72 zu R_{aN} gewählt, d.h. so bemessen, daß die HF-Leistungsstufe 4 beim eingestellten Soll-Wert N* den Maximalwert Nₘₐₓ der Leistung N abgibt. Der Widerstandswert R_{aN} entspricht also dem Nennwert, bei dem die jeweilige Maximalleistung entnommen wird (N* = Nₘₐₓ ). Mit anderen Worten: Bei jedem eingestellten Soll-Wert N* (z.B. N₁* = 300 Watt oder N₂* = 350 Watt) führt ein einziger Lastwiderstand 72 (z.B. mit R_{aN} = 500 Ohm) zur maximalen Leistungsabgabe (im Beispiel N₁ₘₐₓ = N₁* = 300 Watt bzw. N₂ₘₐₓ = N₂* = 350 Watt). Dieser Lastwiderstand 72 ist für alle Soll-Werte N* (z.B. 300 Watt, 350 Watt) annähernd gleich groß.

Ist das HF-Chirurgiegerät 2 in Ordnung, d.h. liegt kein Fehler vor, so wird die Ist-Anzeige 36 (mit einer gewissen Toleranz) der Soll-Anzeige 64 entsprechen. Diese Überprüfung kann optisch durch den Beobachter oder Operateur oder auch hier wieder automatisch durch den Komparator 42 erfolgen. Somit ist also eine Sicherheitsprüfung und zugleich eine pauschale Überwachung des gesamten HF-Chirurgiegeräts 2 möglich. Insbesondere wird dabei der für die Sicherheit des Patienten 18 entscheidende Wert, nämlich die abgegebenen HF-Leistung, herangezogen und angezeigt. Liegt ein nennenswerter Unterschied (N* - N) vor, so ist das ein Zeichen dafür, daß ein Bauelementfehler vorhanden ist. Dies führt automatisch zur Anzeige an den Geräten 36, 64, zur Abgabe eines Alarms an den Einrichtungen 50, 52 und/oder zur Abschaltung des HF-Chirurgiegeräts mit Hilfe des Schalters 58.

In Fig. 1 ist weiterhin eine Schaltung zur Simulation eines Alarms gezeigt. Diese Alarmsimulation beruht auf einer Verschiebung der Schwelle des Komparators 42. Speziell ist hier so vorgegangen, daß bei der Simulation ein zu niedriges Eingangssignal u1 am ersten Eingang 38 des Komparators 42 vorgegeben wird. Dazu ist folgendes vorgesehen: Die Testschaltung 48 kann - nach Betätigung des Testschalters 74 - auch ein zweites Relais 78 ansteuern. Dadurch wird ein Schalter 80 geschlossen, und durch den Widerstand 62 sowie einen weiteren Widerstand 82, der mit dem auf Bezugspotential liegenden Schalter 80 in Serie liegt, wird ein ohmscher Spannungsteiler für den analogen Soll-Wert N* am ersten Komparatoreingang 38 erzeugt. Die beiden Widerstände 62, 82 sind dabei so bemessen, daß die Toleranzgrenze für den Ist-Wert N bei geschlossenem Schalter 80 und normaler Funktion gerade überschritten wird; damit gibt der Komparator 42 ein Freigabesignal f ab. Im vorliegenden Simulationsfall wird es nur für die Auslösung eines Alarms verwendet. Relais 78 und Schalter 80 bilden bevorzugt - im Gegensatz zur zeichnerischen Darstellung - eine Einheit.

Die beiden Widerstände 62, 82 sind hier speziell für eine negative Toleranz eingesetzt. Für den Fall, daß im Normalfall auch eine positive Toleranz des Ist-Werts N der Leistung zugelassen wird, kann ein entsprechender Spannungsteiler 62ʹ, 82ʹ, der durch einen entsprechenden Schalter 80ʹ abgeschaltet wird, am anderen Eingang 40 des Komparators 42 vorgesehen sein. Diese Möglichkeit ist in Fig. 1 gepunktet angedeutet. Die Elemente 62, 78, 80, 82 und 62ʹ, 78ʹ, 80ʹ, 82ʹ stellen somit jeweils eine Hilfsschaltung dar, mit der im Testbetrieb ein Alarm simulierbar ist.

Anstelle der Relais-Schalter-Kombination 78, 80 oder 78ʹ,80ʹ kann auch jeweils ein elektronisches Schaltmittel zur Anwendung kommen, z.B. ein Schalttransistor.

Es soll noch einmal festgehalten werden, daß im Testbetrieb vorwiegend ein Alarm simuliert werden kann. Nach der zuerst erwähnten Möglichkeit war vorgesehen, daß der Soll-Wert N*, z.B. durch den ohmschen Spannungsteiler 62, 82 bei Betätigung des Schalters 80, in einem bestimmten Verhältnis herabgesetzt wird. Die erste Hilfsschaltung 62, 80, 82 ist dabei dem Komparator 42 zugeordnet. Nach der als nächstes erwähnten zweiten Möglichkeit war vorgesehen, daß der Ist-Wert N, z.B. durch Eliminieren des sonst normalerweise immer vorhandenen ohmschen Spannungsteilers 62ʹ, 82ʹ mit Hilfe des zu öffnenden Schalters 80ʹ, um ein bestimmtes Verhältnis erhöht wird. Die zweite Hilfsschaltung 62ʹ, 80ʹ, 82ʹ ist dabei ebenfalls dem Komparator 42 zugeordnet. Eine solche Zuordnung muß nicht sein. So läßt sich nach einer dritten Möglichkeit der Ist-Wert N um ein bestimmtes Verhältnis auch erhöhen durch definierte Vergrößerung der Verstärkung des Leistungsmeßgliedes 30 oder eines anderen Bauelements. Dies ist vorliegend durch einen gepunktet dargestellten Widerstand 86, der von einem Schalter 88 ab- oder zugeschaltet werden kann, am Verstärkungs-Einstellglied 31 des Meßglieds 30 symbolisiert.

Schließlich läßt sich auch ein Alarm simulieren, indem der Soll-Wert N* um einen bestimmten absoluten Betrag U herabgesetzt oder der Ist-Wert N um einen bestimmten Betrag U heraufgesetzt wird. Dies geschieht z.B. dadurch, daß die Komparatarschwelle um einen dementsprechenden Betrag U verschoben wird. dies ist in Figur 1 ebenfalls angedeutet. Dort ist eine Einrichtung 90 zum Umschalten der Komparatorschwelle gezeigt. Ein Umschalter 92, gesteuert von der Testschaltung 48, legt entweder die negative Spannung (-U) einer Gleichspannungsquelle 94 oder Bezugspotential über einen Widerstand 96 an den ersten Eingang 38 des Komparators 42. Alternativ legt ein Umschalter 92ʹ entweder die positive Spannung (+U) einer Gleichspannungsquelle 94ʹ oder Bezugspotential über einen Widerstand 96ʹ an den zweiten Eingang 40 des Komparators 42. Auch die beiden Einrichtungen 90, 90ʹ sind gestrichelt eingezeichnet.

Zur Funktion ist folgendes zu sagen, wobei von der ersten Hilfsschaltung ausgegangen wird (entsprechendes gilt für die anderen Hilfsschaltungen):
1. Im ersten Testfall, beim "Selbsttest", ist das erste Relais 71 angezogen. Die Ist-Wert- und Soll-Wert-Anzeige an den Einrichtungen 36 bzw. 64 müssen im Rahmen bestimmter Toleranzen übereinstimmen, und der Komparator 42 darf keinen Alarm melden. In diesem Fall ist das HF-Chirurgiegerät 2 frei von einem ersten Fehler. Dabei ist die Prüfung nicht unter Einschluß des Patienten 18 vorgenommen worden.
2. Im zweiten Testfall, bei der "Alarm-Simulation", sind beide Relais 71, 78 angezogen. In diesem Fall muß der Komparator 42 einen Alarm auslösen. Dadurch kann geprüft werden, ob die Überwachungseinrichtung funktionsfähig ist.

Der gesamte Testablauf läßt sich automatisieren in der Weise, daß durch Betätigen der Testtaste 74 nacheinander der erste und zweite Test (d.h. "Selbsttest" und "Alarm-Simulation") ablaufen und automatisch ausgewertet werden. Das Gerät 2 ist fehlerfrei, wenn im ersten Testfall der Komparator 42 nicht anspricht, dagegen aber im zweiten Testfall anspricht. Umgekehrt muß ein Gerätefehler vorliegen, wenn der Komparator 42 im ersten Testfall anspricht oder im zweiten Testfall nicht anspricht. In diesem Fall erfolgt eine Alarmabgabe und/oder das Gerät 2 wird gesperrt. Der automatische Testablauf bringt außerdem den Vorteil, daß der Lastwiderstand 72 nur kurzzeitig belastet wird.

## Patentansprüche

1. Überwachungsschaltung in einem HF-Chirurgiegerät, das eine HF-Leistungsstufe, eine Steuerschaltung für deren Ansteuerung, ein Einstellorgan (8) zum Einstellen eines Sollwertes (N*) der über Elektroden (14, 18) abgegebenen HF-Leistung und eine Einrichtung (42) zum Vergleich des Sollwertes (N*) mit einem Istwert (N) umfaßt, **dadurch gekennzeichnet,** daß zwischen dem Ausgang der HF-Leistungsstufe (4) und den Elektroden (14, 18) ein Spannungsmeßglied (26) und ein Strommeßglied (28) angeordnet und mit einem analogen Leistungsmesser (30) zum Messen des Istwertes (N) der abgegebenen HF-Leistung verbunden sind und daß ein Lastwiderstand (72) mit vorgebbarem Widerstandswert (Rₐ) an den Ausgang der HF-Leistungsstufe (4) anstelle der Elektroden (14, 18) für eine Sicherheitsprüfung des HF-Chirurgiegerätes (2) mit Hilfe eines Schalters (70) anschaltbar ist.

2. Überwachungsschaltung nach Anspruch 1, **dadurch gekennzeichnet,** daß der Widerstandswert (Rₐ) des Lastwiderstandes (72) der Nennwert (R_{aN}) ist, bei dem die HF-Leistungsstufe (4) den eingestellten Sollwert (N₁*, N₂*) der Leistung abgibt.

3. Überwachungsschaltung nach Anspruch 1 oder 2, **dadurch gekennzeichnet,** daß für mehrere Leistungssollwerte (N₁*, N₂*) der Lastwiderstand (72) denselben Widerstandswert (R_{aN}) besitzt.

4. Überwachungsschaltung nach einem der Ansprüche 1, 2 oder 3, **dadurch gekennzeichnet,** daß der Schalter ein von einer Testschaltung (48) betätigbarer Umschalter (70) ist.

5. Überwachungsschaltung nach einem der Ansprüche 1 bis 4, **dadurch gekennzeichnet,** daß der Sollwert (N*) und der Istwert (N) den Eingängen (38, 40) eines Komparators (42) zugeführt sind.

6. Überwachungsschaltung nach Anspruch 5, **dadurch gekennzeichnet,** daß der Komparator (42) so ausgebildet ist, daß er bei einem nennenswerten Unterschied (N-N*) zwischen zugeführtem Istwert (N) und zugeführtem Sollwert (N*) ein Freigabesignal (f) zur Freigabe eines Alarms (50, 52) und/oder einer Geräteabschaltung (58) abgibt.

7. Überwachungsschaltung nach Anspruch 5 oder 6, **dadurch gekennzeichnet,** daß wenigstens an einem Eingang des Komparators (42) eine Hilfsschaltung (62, 80, 82; 62', 80', 82'; 90, 96; 90', 96') vorgesehen ist, mit der wenigstens ein Eingangswert (Sollwert oder Istwert) so weit veränderbar ist, daß im Testbetrieb ein Alarm (50, 52) simulierbar ist.

8. Überwachungsschaltung nach Anspruch 1, **dadurch gekennzeichnet,** daß der Leistungsmesser (26, 28, 30) ein Verstärkungs-Einstellglied (31) aufweist, das mit einer Hilfsschaltung (86, 88) zur Einstellung des Istwertes (N) verbunden ist.

9. Überwachungsschaltung nach Anspruch 7 oder 8, **dadurch gekennzeichnet,** daß die Hilfsschaltung (62, 80, 82; 62', 80', 82'; 86, 88; 90-96; 90'-96') so ausgebildet ist, daß mit ihr das dem Sollwert (N) entsprechende, dem ersten Eingang (38) des Komparators (42) zugeführte erste Eingangssignal (u1) auf einen niedrigeren Wert einstellbar ist als das bei ungestörtem Betrieb des HF-Chirurgiegeräts (2) dem Istwert (N) entsprechende, dem zweiten Eingang (40) des Komparators (42) zugeführte zweite Eingangssignal (u2), und daß der Komparator (42) ein Freigabesignal (f) für den simulierten Alarm (50, 52) abgibt, sobald das erste Eingangssignal (u1) kleiner als das zweite Eingangssignal (u2) ist.

10. Überwachungsschaltung nach Anspruch 7 oder 9, **dadurch gekennzeichnet,** daß die Hilfsschaltung (62, 80, 82; 62', 80', 82'; 90-96; 90'-96') über einen Schalter (80, 80'; 92, 92') dem Komparator (42) zuschaltbar ist.

11. Überwachungsschaltung nach einem der Ansprüche 1 bis 10, **dadurch gekennzeichnet,** daß der Leistungsmesser (26, 28, 30) ein Multiplizierglied (30) umfaßt, dem von einem Transformator (26) die gemessene HF-Spannung (U) und von einem Transformator (28) der gemessene HF-Strom (I) zuführbar sind.

12. Überwachungsschaltung nach einem der Ansprüche 1 bis 11, **dadurch gekennzeichnet,** daß ein Schalter (74) vorgesehen ist, bei dessen Betätigung automatisch ein Testprogramm einleitbar ist, bei dem der Lastwiderstand (72) an den Ausgang der HF-Leistungsstufe (4) angeschaltet, eine Überprüfung der Funktion des HF-Chirurgiegeräts (2) durch Vergleich von Istwert (N) und Sollwert (N*) vorgenommen und ein Alarm (50, 52) simuliert wird.

## Claims

1. Monitoring circuit in a HF surgical apparatus, which comprises a HF output stage, a control circuit for its control, a setting part (8) for setting a desired value (N*) of the HF output delivered by way of electrodes (14, 18), and a device (42) for comparing the desired value (N*) with an actual value (N), characterized in that: between the output of the HF output stage (4) and the electrodes (14, 18) a voltage measuring element (26) and a current measuring element (28) are arranged and are connected to an analogue output meter (30) for measuring the actual value (N) of the HF output delivered, and in that: a load resistance (72) with specifiable resistance value (Rₐ) can be connected with the aid of a switch (70) to the output of the HF output stage (4) in place of the electrodes (14, 18) for a safety test of the HF surgical apparatus (2).

2. Monitoring circuit according to claim 1, characterized in that the resistance value (Rₐ) of the load resistance (72) is the nominal value (R_{aN}) at which the HF output stage (4) delivers the set desired value (N₁*, N₂*) of the output.

3. Monitoring circuit according to claim 1 or 2, characterized in that the load resistance (72) has the same resistance value (R_{aN}) for several desired output values (N₁*, N₂*).

4. Monitoring circuit according to one of claims 1, 2 or 3, characterized in that the switch is a changeover switch (70) which can be actuated by a test circuit (48).

5. Monitoring circuit according to one of claims 1 to 4, characterized in that the desired value (N*) and the actual value (N) are supplied to the inputs (38, 40) of a comparator (42).

6. Monitoring circuit according to claim 5, characterized in that the comparator (42) is constructed in such a way that, with a considerable difference (N-N*) between the supplied actual value (N) and the supplied desired value (N*), it delivers a release signal (f) for the release of an alarm (50, 52) and/or a disconnection (58) of the apparatus.

7. Monitoring circuit according to claim 5 or 6, characterized in that at least at one input of the comparator (42) there is provided an auxiliary circuit (62, 80, 82; 62', 80', 82'; 90, 96; 90', 96'), with which at least one input value (desired value or actual value) can be altered so much that in test operation an alarm (50, 52) can be simulated.

8. Monitoring circuit according to claim 1, characterized in that the output meter (26, 28, 30) has an amplification setting element (31), which is connected to an auxiliary circuit (86, 88) for setting the actual value (N).

9. Monitoring circuit according to claim 7 or 8, characterized in that the auxiliary circuit (62, 80, 82; 62', 80', 82'; 86, 88; 90-96; 90'-96') is constructed in such a way that the first input signal (u1) corresponding with the desired value (N*) and supplied to the first input (38) of the comparator (42) can be set by it to a lower value than the second input signal (u2) corresponding with the actual value (N) with undisturbed operation of the HF surgical apparatus (2) and supplied to the second input (40) of the comparator (42), and in that the comparator (42) delivers a release signal (f) for the simulated alarm (50, 52) as soon as the first input signal (u1) is smaller than the second input signal (u2).

10. Monitoring circuit according to claim 7 or 9, characterized in that the auxiliary circuit (62, 80, 82; 62', 80', 82'; 90-96; 90'-96') can be connected to the comparator (42) by way of a switch (80, 80'; 92,92').

11. Monitoring circuit according to one of claims 1 to 10, characterized in that the output meter (26, 28, 30) comprises a multiplying element (30), to which there can be supplied from a transformer (26) the measured HF voltage (U) and from a transformer (28) the measured HF current (I).

12. Monitoring circuit according to one of claims 1 to 11, characterized in that a switch (74) is provided, with the actuation of which a test program can automatically be introduced, with which the load resistance (72) is connected to the output of the HF output stage (4), a testing of the function of the HF surgical apparatus (2) through comparison of actual value (N) and desired value (N*) is undertaken and an alarm (50, 52) is simulated.

## Revendications

1. Circuit de contrôle dans un appareil chirurgical à haute fréquence, qui comprend un étage de puissance haute fréquence, un circuit de commande pour son attaque, un organe de réglage (8) pour ajuster une valeur de consigne (N*) de la puissance haute fréquence est débitée par l'intermédiaire d'électrodes (14, 18) et un dispositif (42) de comparaison entre la valeur de consigne (N*) et une valeur instantanée (N), caractérisé en ce que, entre la sortie de l'étage de puissance haute fréquence (4) et les électrodes (14, 18), sont disposés un élément de mesure de la tension (26) et un élément de mesure du courant (28) et sont reliés à un wattmètre analogique (30) pour la mesure de la valeur instantanée (N) de la puissance haute fréquence fournie, et en ce qu'une résistance de charge (72), à valeur de résistance définissable à l'avance (Rₐ) est susceptible d'être reliée, à l'aide d'un interrupteur (70), à la sortie de l'étage de puissance haute fréquence (4), à la place des électrodes (14, 18), pour un test de sécurité de l'appareil chirurgical à haute fréquence (2).

2. Circuit de contrôle selon la revendication 1, caractérisé en ce que la valeur de la résistance (Rₐ) de la résistance de charge (72) est la valeur nominale (R_{aN}) pour laquelle l'étage de puissance haute fréquence (4) délivre la valeur de consigne de puissance (N₁*, N₂*) ajustée.

3. Circuit de contrôle selon la revendication 1 ou 2, caractérisé en ce que, pour plusieurs valeurs de consigne de la puissance (N₁*, N₂*), la résistance de charge (72) possède la même valeur de résistance (R_{aN}).

4. Circuit de contrôle selon l'une quelconque des revendications 1, 2 ou 3, caractérisé en ce que l'interrupteur est un commutateur (70) qui peut être commandé par un circuit de test (48).

5. Circuit de contrôle selon l'une quelconque des revendications 1 à 4, caractérisé en ce que la valeur de consigne (N*) et la valeur instantanée (N) sont appliquées aux entrées (38, 40) d'un comparateur (42).

6. Circuit de contrôle selon la revendication 5, caractérisé en ce que le comparateur (42) est réalisé de telle façon qu'il délivre, pour une différence notable (N-N*) entre la valeur instantanée appliquée (N) et la valeur de consigne appliquée (N*), un signal de validation pour l'enclenchement d'une alarme (50, 52) et/ou d'une débranchement de l'appareil (58).

7. Circuit de contrôle selon les revendications 5 ou 6, caractérisé en ce qu'il est prévu au moins un circuit auxiliaire (62, 80, 82 ; 62', 80', 82' ; 90, 96 ; 90', 96') à une entrée du comparateur (42), avec lequel au moins une valeur d'entrée (valeur de consigne ou valeur effective) est susceptible d'être modifiée, ou qu'une alarme (50,52) puisse être simulée lors d'une opération de test.

8. Circuit de contrôle selon la revendication 1, caractérisé en ce que le wattmètre (26, 28, 30) comporte un élément de réglage d'amplification (31), qui est relié à un étage auxiliaire (86, 88) pour le réglage de la valeur instantanée (N).

9. Circuit de contrôle selon les revendications 7 ou 8, caractérisé en ce que le montage auxiliaire (62, 80, 82 ; 62', 80', 82' ; 86, 88 ; 90-96 ; 90'-96') est formé de sorte que, avec lui, le premier signal d'entrée (u1) appliqué à la première entrée (38) du comparateur (42) pour une valeur de consigne (N*) correspondante, est ajustable à une valeur faible, comme le second signal d'entrée (u2) appliqué à la seconde entrée (40) du comparateur (42) pour une valeur effective pouvant être (N) correspondante, pour un bon fonctionnement de l'appareil chirurgical et en ce que le comparateur (42) délivre un signal de validation pour l'alarme simulée, aussitôt que le premier signal d'entrée (u1) est plus petit que le second signal d'entrée (u2).

10. Circuit de contrôle selon les revendications 7 ou 9, caractérisé en ce que le circuit auxiliaire (62, 80, 82 ; 62', 80', 82' ; 86, 88 ; 90-96 ; 90'-96') est susceptible d'être relié au comparateur (42) par l'intermédiaire d'un interrupteur (80, 80' ; 92, 92').

11. Circuit de contrôle selon l'une quelconque des revendications 1 à 10, caractérisé en ce que le wattmètre (26, 28, 30) comporte un élément multiplicateur (30), auquel sont susceptibles d'être reliés la tension haute fréquence mesurée par un transformateur (26) et le courant haute fréquence (I) mesuré par un transformateur (28).

12. Circuit de contrôle selon l'une des revendications 1 à 11, caractérisé en ce qu'il est prévu un interrupteur (74), par la commande duquel peut être initié automatiquement un programme de test, à l'aide duquel, la résistance de charge (72) est reliée à la sortie de l'étage de puissance haute fréquence (4), une vérification du fonctionnement de l'appareil chirurgical haute fréquence (2) est operée par une comparaison de la valeur instantanée (N) et de la valeur de consigne (N*) et une alerte (50, 52) est simulée.
